# EUROPEAN PATENT APPLICATION

(11) **EP 4 516 780 A1**
(43) Date of publication of application: **05.03.2025**
(21) Application number: 23796252.7
(22) Date of filing: 20.04.2023
(51) Int. Cl.: C07D 257/02, C07D 403/12, C07F 7/00

(54) **METHOD FOR PRODUCING RADIOACTIVE METAL COMPLEX**

(30) Priority: 27.04.2022 JP 2022073792
(71) Applicant: Nihon Medi-Physics Co., Ltd., Tokyo 136-0075 (JP); Kyoto University, Kyoto-shi, Kyoto 606-8501 (JP)
(72) Inventor: ICHIKAWA, Hiroaki, Tokyo 136-0075 (JP); KISHIMOTO, Satoshi, Tokyo 136-0075 (JP); OTSUKA, Yuta, Tokyo 136-0075 (JP); IMAI, Tomoyuki, Tokyo 136-0075 (JP); TAKAYA, Hikaru, Kyoto-shi, Kyoto 606-8501 (JP)
(74) Representative: TBK
(86) International application number: PCT/JP2023/015836
(87) International publication number: WO 2023/210510

(57) **Abstract**

A method for producing a radioactive metal complex, including a complex forming step of reacting a radioactive metal nuclide with a ligand compound represented by the following formula (1) in a reaction liquid containing water and a buffer to form a radioactive metal complex, the buffer containing one or more water-soluble organic compounds having a sulfo group or a carboxy group, and the reaction liquid being irradiated with microwave while cooling the reaction liquid in the complex forming step. The radioactive metal nuclide is preferably ⁶⁸Ga, ⁸⁹Zr, ⁹⁰Y, ¹¹¹In, ¹⁷⁷Lu, or ²²⁵Ac.

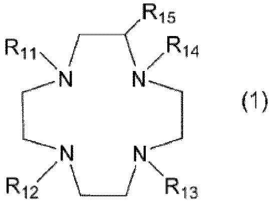

## Description

### Technical Field

The present invention relates to a method for producing a radioactive metal complex.

### Background Art

Studies for efficiently synthesizing a radioactive metal complex in which a ligand compound is coordinated to a radioactive metal nuclide have been conducted for the purpose of use in reagents and diagnostics for detection of target molecules or pharmaceuticals for treatment of diseases. One of such studies is to irradiate a radioactive metal nuclide with microwave when performing a reaction of coordinating a ligand compound to the radioactive metal nuclide (hereinafter, it is also referred to as a "radioactive metal complexation reaction".).

Patent Literature 1 describes a method for producing a radioactive metal complex labeled with ²²⁵Ac. The same literature describes that heating by microwave irradiation is preferable when heating is required at the time of performing a radioactive metal complexation reaction between a ligand compound and ²²⁵Ac.

Non Patent Literature 1 describes a method for producing a radioactive metal complex including ⁶⁸Ga and DOTATOC as a ligand compound. The same literature describes that an intended ⁶⁸Ga complex is obtained in a shorter time by heating to 90°C by irradiation of microwave during a radioactive metal complexation reaction as compared with the case of heating with a block heater.

Non Patent Literature 2 describes a method for producing a radioactive metal complex including ⁸⁹Zr and TRITA as a ligand compound. The same literature describes that an intended ⁸⁹Zr complex is obtained in a shorter time and in a higher yield by heating to 180°C by irradiation of microwave during a radioactive metal complexation reaction as compared with the case of using an ordinary heating method.

### Citation List

### Patent Literature

Patent Literature 1: US 2015/0157742 A

### Non Patent Literature

Non Patent Literature 1: I. Velikyan et al., Bioconjugate Chem. 2004, 15, 554-560
Non Patent Literature 2: D. N. Pandya et al., Inorg. Chem. 2020, 59, 17473-17487

### Summary of Invention

### Technical Problem

In Patent Literature 1, Non Patent Literature 1, and Non Patent Literature 2, detailed conditions for microwave irradiation in a radioactive metal complexation reaction have not been studied. For this reason, the reaction acceleration effect by the microwave irradiation is not maximized, and there remains room for improvement in the reaction efficiency of the radioactive metal complexation reaction.

Therefore, an object of the present invention is to more efficiently progress the radioactive metal complexation reaction.

The present invention relates to a method for producing a radioactive metal complex, including:
a complex forming step of reacting a radioactive metal nuclide
with a ligand compound represented by the following formula (1) in a reaction liquid containing water and a buffer to form a radioactive metal complex,
the buffer containing one or more water-soluble organic compounds having a sulfo group or a carboxy group, and
the reaction liquid being irradiated with microwave while the reaction liquid is cooled in the complex forming step. 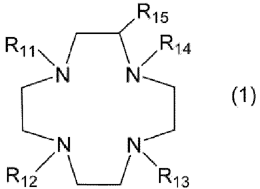
wherein R₁₁, R₁₂, and R₁₃ are each independently a group consisting of - (CH₂)ₚCOOH, -(CH₂)ₚC₅H₄N, -(CH₂)ₚPO₃H₂, or -(CH₂)ₚCONH₂, one of R₁₄ and R₁₅ is a group consisting of a hydrogen atom, -(CH₂)ₚCOOH, -(CH₂)ₚC₅H₄N, -(CH₂)ₚPO₃H₂, - (CH₂)ₚCONH₂ or -(CHCOOH)(CH₂)ₚCOOH, and the other is a group consisting of - (CH₂)ₚCOOH, -(CH₂)ₚC₅H₄N, -(CH₂)ₚPO₃H₂, or -(CH₂)ₚCONH₂, or a reactive atomic group for linking to a targeting agent or a group linking to a targeting agent, and each p is independently an integer of 0 or more and 3 or less.

According to the present invention, there is provided a method for producing a radioactive metal complex capable of more efficiently progressing a radioactive metal complexation reaction.

### Description of embodyments

Hereinafter, a method for producing a radioactive metal complex of the present invention will be described based on preferred embodiments thereof. The production method of the present invention includes a complex forming step of reacting a radioactive metal nuclide with a ligand compound represented by formula (1) described later in a reaction liquid to form a radioactive metal complex.

The radioactive metal complex is a compound in which a radioactive metal atom is bonded to a ligand compound by a combination of a covalent bond, an ionic bond, or the like in addition to a coordination bond, and also includes a compound to which a reactive atomic group or a targeting agent described later is further bonded.

In the present specification, forming a complex of a radioactive metal ion with a ligand compound and labeling a ligand compound with a radioactive metal ion are synonymous, and complexation efficiency and a labeling rate are synonymous.

From the viewpoint of increasing the labeling rate, the radioactive metal nuclide used in the complex forming step is preferably used in the form of a compound capable of being ionized in water, and more preferably used in the form of a metal ion (hereinafter, these embodiments are also collectively referred to as a "radioactive metal source".). As the radioactive metal source, for example, a radioactive metal ion-containing liquid in which radioactive metal ions are dissolved or dispersed in a solvent mainly composed of water can be used.

The radioactive metal nuclide contained in a radioconjugate of the present invention is a radionuclide that emits α rays, a radionuclide that emits β rays, a radionuclide that emits positrons, or a radionuclide that emits γ rays. When the radioconjugate of the present invention is used for cancer treatment, it is preferable to use a radionuclide that emits α rays or a radionuclide that emits β rays. Also, when the radioconjugate of the present invention is used for diagnosis or detection of cancer, it is preferable to use a radionuclide that emits positrons or a radionuclide that emits γ rays. Examples of the radionuclide that emits α rays include ²¹²Bi, ²¹³Bi, ²²⁵Ac, and ²²⁷Th. Also, examples of the radionuclide that emits β rays include ⁶⁴Cu, ⁹⁰Y, or ¹⁷⁷Lu. Further, examples of the radionuclide that emits positrons include ⁶⁴Cu, ⁶⁸Ga, ⁸⁶Y, and ⁸⁹Zr. Furthermore, examples of the radionuclide that emits γ rays include ^{99m}Tc or ¹¹¹In. The radioactive metal nuclide contained in the radioconjugate of the present invention is more preferably ²²⁵Ac, ⁹⁰Y, ¹⁷⁷Lu, or ⁸⁹Zr.

The concentration of the radioactive metal nuclide in the reaction liquid is preferably 1 nmol/L or more and 10,000 nmol/L or less, more preferably 1 nmol/L or more and 5,000 nmol/L or less, further preferably 1 nmol/L or more and 1,000 nmol/L or less, and still more preferably 1 nmol/L or more and 500 nmol/L or less at the start of the reaction between the radioactive metal nuclide and the ligand compound (hereinafter, it is also referred to as "at the start of the complex forming step" or "at the start of the reaction".).

The radioactive metal nuclide is, for example, dissolved in a suitable solvent after production and stored in a state of solution, and the required amount can be taken out from this solution when necessary and used for the present invention. Hereinafter, the solution for storing the radioactive metal nuclide is also referred to as a "bulk solution".

As radionuclide decay proceeds, the bulk solution volume required to use the desired radioactivity for the complexation reaction increases. This bulk solution is mixed with a non-radioactive metal used at the time of producing the radioactive metal nuclide. Therefore, if the volume of the bulk solution to be used increases, the amount of the non-radioactive metal mixed in the complexation reaction also increases, which causes a decrease in the yield of the complex forming reaction. Thus, the radionuclide is preferably used immediately after production.

The ligand compound used in the complex forming step has a structure represented by the following formula (1).

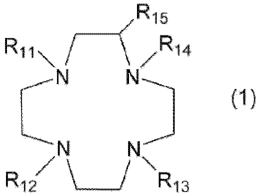

In the formula (1), R₁₁, R₁₂, and R₁₃ are each independently a group consisting of -(CH₂)ₚCOOH, -(CH₂)ₚC₅H₄N, -(CH₂)ₚPO₃H₂, or -(CH₂)ₚCONH₂.

Each p above is independently an integer of 0 or more and 3 or less.

In the formula (1), one of R₁₄ and R₁₅ is a group consisting of a hydrogen atom, -(CH₂)ₚCOOH, -(CH₂)ₚC₅H₄N, -(CH₂)ₚPO₃H₂, -(CH₂)ₚCONH₂ or - (CHCOOH)(CH₂)ₚCOOH.

In the formula (1), the other of R₁₄ and R₁₅ is a group consisting of - (CH₂)ₚCOOH, -(CH₂)ₚC₅H₄N, -(CH₂)ₚPO₃H₂, or -(CH₂)ₚCONH₂, or a reactive atomic group for linking to a targeting agent or a group linking to a targeting agent.

Each p above is independently an integer of 0 or more and 3 or less.

Details of the targeting agent, and the reactive atomic group for linking to a targeting agent or the group linking to a targeting agent will be described later.

More specifically, the ligand compound used in the complex forming step more preferably contains one compound shown below or a structure derived from the compound. The ligand compound used in the complex forming step is preferably water-soluble.

DOTA (1,4,7,10-Tetraazacyclododecane-1,4,7,10-tetraacetic acid)
DOTMA ((1R,4R,7R,10R)-a,a',a",a‴-tetramethyl-1,4,7,10-tetraazacyclododecane-1,4,7,10-tetraacetic acid)
DOTAM (1,4,7,10-tetrakis(carbamoylmethyl)-1,4,7,10-tetraazacyclododecane)
DOTA-GA (α-(2-Carboxyethyl)-1,4,7,10-tetraazacyclododecane-1,4,7,10-tetraacetic acid)
DOTP (((1,4,7,10-Tetraazacyclododecane-1,4,7,10-tetrayl)tetrakis(methylene))tetraphosphonic acid)
DOTMP (1,4,7,10-Tetraazacyclododecane-1,4,7,10-tetrakis(methylenephosphonic acid))
DOTA-4AMP (1,4,7,10-tetraazacyclododecane-1,4,7,10-tetrakis(acetamidomethylenephosphonic acid)
DO2P (Tetraazacyclododecane dimethanephosphonic acid)

From the viewpoint of increasing the yield of the intended radioactive metal complex, the concentration of the ligand compound in the reaction liquid is preferably 1 µmol/L or more and 1,000 µmol/L or less, more preferably 1 µmol/L or more and 900 µmol/L or less, further preferably 1 µmol/L or more and 600 µmol/L or less, and still more preferably 1 µmol/L or more and 500 µmol/L or less at the start of the complex forming step.

The reaction liquid in the complex forming step is an aqueous reaction liquid containing water and a buffer.

As the water, water commonly used in the present technical field can be employed, and for example, distilled water or ion-exchanged water can be used.

The buffer contains a water-soluble organic compound having a predetermined structure. The water-soluble organic compound is an organic compound that dissolves in water, and is a compound different from the above-described ligand compound. Therefore, the water-soluble organic compound in the specification of the present application is not included in the ligand compound.

In the following description, a water-soluble organic compound that has a predetermined structure and is not included in the ligand compound and the organic solvent is also referred to as a "second organic compound".

The second organic compound contained in the reaction liquid has a specific functional group in the structure thereof. Specifically, as an embodiment of the second organic compound, the second organic compound has a sulfo group in the structure. The sulfo group is a monovalent functional group represented by "-SO₃H" or "-SO₃⁻". The second organic compound in this form preferably has one or two sulfo groups, i.e., is preferably a monosulfonic acid or a disulfonic acid, from the viewpoint of being easily available, and increasing the yield of the radioactive metal complex while reducing the production cost.

When having a sulfo group in the structure, the total carbon number of the second organic compound is preferably 4 or more and 10 or less, and more preferably 6 or more and 8 or less.

Also, when having a sulfo group in the structure, the second organic compound preferably has a hetero atom in the structure, preferably has at least a nitrogen atom in the structure, further preferably has a cyclohexane ring or a heterocyclic ring in the structure, still preferably has two nitrogen atoms or contains a saturated heterocyclic ring having a nitrogen atom and an oxygen atom in the structure, and still more preferably contains a morpholine ring or a piperazine ring in the structure.

Moreover, when having a sulfo group in the structure, it is also preferable to have an alkanesulfonic acid group in the structure, it is also preferable that the alkanesulfonic acid group is bonded to a hetero atom, and it is more preferable to have an aminoalkanesulfonic acid in the structure.

In any of the cases described above, the second organic compound having a sulfo group in the structure is preferably a zwitterionic compound, and more preferably an aminoalkanesulfonic acid derivative.

Examples of the second organic compound having one sulfo group in the structure include linear amine monosulfonic acids such as N-tris(hydroxymethyl)methyl-2-aminoethanesulfonic acid (TES); monosulfonic acids having a morpholine ring, such as 2-morpholinoethanesulfonic acid (MES) and 3-morpholinopropanesulfonic acid (MOPS); monosulfonic acids having a piperazine ring, such as 2-[4-(2-hydroxyethyl)-1-piperazinyl]-ethanesulfonic acid (HEPES); and/or salts thereof; and the like.

Examples of the second organic compound having two sulfo groups in the structure include disulfonic acids having a piperazine ring in the structure, such as 2-[4-(2-hydroxyethyl)-1-piperazinyl]-ethanesulfonic acid (HEPES) and piperazine-1,4-bis(2-ethanesulfonic acid) (PIPES), and/or salts thereof, and the like.

Examples of a counter ion of the second organic compound having a sulfo group in the structure include alkali metal ions such as sodium and potassium, cations such as primary to quaternary ammonium such as ammonium and tetramethylammonium salt, and anions such as halogen such as chlorine.

As another embodiment of the second organic compound contained in the reaction liquid, the second organic compound has a carboxy group in the structure. The carboxy group is a monovalent functional group represented by "-COOH" or "-COO⁻". The second organic compound in this form preferably has one or two carboxy groups, and is preferably a monocarboxylic acid or a dicarboxylic acid, from the viewpoint of being easily available, and increasing the yield of the radioactive metal complex while reducing the production cost.

When having a carboxy group in the structure, the total number of carbon atoms of the second organic compound is preferably 2 or more and 10 or less, and more preferably 2 or more and 8 or less.

Also, when having a carboxy group in the structure, the second organic compound is preferably a saturated or unsaturated aliphatic carboxylic acid or aromatic carboxylic acid, and more preferably a saturated aliphatic carboxylic acid.

Examples of the second organic compound having one carboxy group in the structure include linear aliphatic monocarboxylic acids such as acetic acid and lactic acid; aromatic monocarboxylic acids such as benzoic acid and salicylic acid; and/or salts thereof; and the like.

Examples of the second organic compound having two carboxy groups in the structure include linear aliphatic dicarboxylic acids such as malonic acid and tartaric acid, aromatic dicarboxylic acids such as phthalic acid; and/or salts thereof; and the like.

Examples of a counter ion of the second organic compound having a carboxy group in the structure include alkali metal ions such as sodium and potassium, and cations such as primary to quaternary ammonium such as ammonium and tetramethylammonium salts.

Among them, as the second organic compound having a sulfo group or a carboxy group in the structure, one or more selected from acetic acid, phthalic acid, malonic acid, or 2-[4-(2-hydroxyethyl)-1-piperazinyl]-ethanesulfonic acid, N-tris(hydroxymethyl)methyl-2-aminoethanesulfonic acid or 2-morpholinoethanesulfonic acid, and salts thereof are further preferably used, and one or more selected from acetic acid or 2-[4-(2-hydroxyethyl)-1-piperazinyl]-ethanesulfonic acid, and salts thereof are still more preferably used.

The reaction liquid containing a suitable second organic compound can be used in the complex forming step in a state of being prepared in advance as an aqueous solution containing these organic compounds. The reaction liquid may be a buffer solution that exhibits a pH buffering action or may be a liquid that does not exhibit a pH buffering action in the complex forming step.

From the viewpoint of further enhancing the labeling efficiency, the concentration of the second organic compound in the reaction liquid is preferably 0.01 mol/L or more and 2.0 mol/L or less, and more preferably 0.1 mol/L or more and 1.0 mol/L or less at the start of the complex forming step.

The reaction liquid may contain a stabilizer in addition to water and a buffer. The stabilizer contained in the reaction liquid has a structure represented by the following formula (2) or a salt thereof. The stabilizers may be used singly or in combination of two or more types. Depending on the type of the radioactive metal nuclide, by containing such a stabilizer in the reaction liquid, adsorption of the radioactive metal or the intended radioactive metal complex to the inner wall of the reaction vessel can be suppressed even when the radioactivity at the start of the reaction (charged radioactivity) is increased for the purpose of commercial production or the like. As a result, the yield of the intended radioactive metal complex can be increased.

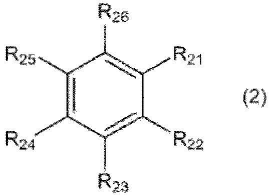

In the formula (2), R₂₁ is -COOH, -CH₂COOH, -CH₂OH, -COOR₂₈, -CONH₂ or -CONHR₂₈.

In the formula (2), one or more and three or less groups of R₂₂ to R₂₆ are a hydroxy group (-OH), and other groups are a hydrogen atom.

In the formula (2), when R₂₁ includes R₂₈, R₂₈ is substituted or unsubstituted alkyl, substituted or unsubstituted aryl, or substituted or unsubstituted alkylaryl. Examples of the substituent that may be substituted with R₂₈ include a halogen atom, a saturated or unsaturated alkyl group, a hydroxy group, a formyl group, a carboxy group, an acyl group, an amino group, a nitro group, an ester group, an isothiocyanate group, a thioxo group, a cyano group, an amide group, an imide group, a phosphate group, a phenyl group, a benzyl group, a pyridyl group, and the like. One of these substituents may be substituted alone, or two or more of these substituents may be combined and substituted.

R₂₈ may be linear or branched, and may be saturated or unsaturated.

The total carbon number of R₂₈ is preferably 1 or more and 10 or less, and more preferably 1 or more and 8 or less.

When the stabilizer represented by the formula (2) is used as a salt, examples of a counter ion include alkali metal ions such as sodium and potassium, and cations such as primary to quaternary ammonium such as ammonium and tetramethylammonium salts.

Examples of the structure of the stabilizer represented by the formula (2) include, but are not limited to, structures represented by any of the following formulas (2a) to (2g).

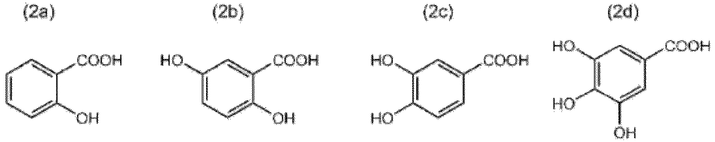

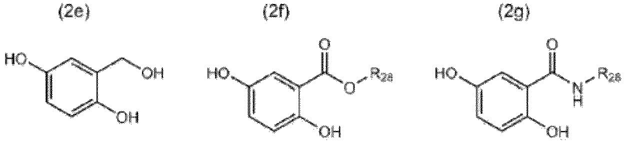

In the formula (2g), R₂₈ is substituted or unsubstituted alkyl, substituted or unsubstituted aryl, or substituted or unsubstituted alkylaryl. Examples of the substituent that may be substituted with R₂₈ include a halogen atom, a saturated or unsaturated alkyl group, a hydroxy group, a formyl group, a carboxy group, an acyl group, an amino group, a nitro group, an ester group, an isothiocyanate group, a thioxo group, a cyano group, an amide group, an imide group, a phosphate group, a phenyl group, a benzyl group, a pyridyl group, and the like. One of these substituents may be substituted alone, or two or more of these substituents may be combined and substituted.

An embodiment of the stabilizer represented by the formula (2) includes forms in which R₂₁ is a carboxy group (-COOH). That is, the stabilizer in this form is hydroxybenzoic acid.

Examples of the hydroxybenzoic acid represented by the formula (2) include monohydroxybenzoic acid, dihydroxybenzoic acid, and trihydroxybenzoic acid.

Examples of the monohydroxybenzoic acid include the following forms.
· 2-Hydroxybenzoic acid (salicylic acid): In the formula (2), R₂₁ is -COOH, R₂₂ is -OH, and all of R₂₃ to R₂₆ are a hydrogen atom. This form corresponds to the formula (2a).
· 3-Hydroxybenzoic acid: In the formula (2), R₂₁ is -COOH, R₂₃ is -OH, and all of R₂₂ and R₂₄ to R₂₆ are a hydrogen atom.
· 4-Hydroxybenzoic acid: In the formula (2), R₂₁ is -COOH, R₂₄ is -OH, and all of R₂₂, R₂₃, R₂₅ and R₂₆ are a hydrogen atom.

Examples of the dihydroxybenzoic acid include the following forms.
· 2,3-Dihydroxybenzoic acid (2-pyrocatechuic acid): In the formula (2), R₂₁ is -COOH, both R₂₂ and R₂₃ are -OH, and all of R₂₄ to R₂₆ are a hydrogen atom.
· 2,4-Dihydroxybenzoic acid (β-resorcinic acid): In the formula (2), R₂₁ is - COOH, both R₂₂ and R₂₄ are -OH, and all of R₂₃, R₂₅ and R₂₆ are a hydrogen atom.
· 2,5-Dihydroxybenzoic acid (gentisic acid): In the formula (2), R₂₁ is -COOH, both R₂₂ and R₂₅ are -OH, and all of R₂₃, R₂₄ and R₂₆ are a hydrogen atom. This form corresponds to the formula (2b).
· 2,6-Dihydroxybenzoic acid (γ-resorcinic acid): In the formula (2), R₂₁ is - COOH, both R₂₂ and R₂₆ are -OH, and all of R₂₃, R₂₄ and R₂₅ are a hydrogen atom.
· 3,4-Dihydroxybenzoic acid (protocatechuic acid): In the formula (2), R₂₁ is - COOH, both R₂₃ and R₂₄ are -OH, and all of R₂₂, R₂₅ and R₂₆ are a hydrogen atom. This form corresponds to the formula (2c).
· 3,5-Dihydroxybenzoic acid (α-resorcinic acid): In the formula (2), R₂₁ is - COOH, both R₂₃ and R₂₅ are -OH, and all of R₂₂, R₂₄ and R₂₆ are a hydrogen atom.

Examples of the trihydroxybenzoic acid include, but are not limited to, the following forms.
· 3,4,5-Trihydroxybenzoic acid (gallic acid): In the formula (2), R₂₁ is -COOH, all of R₂₃ to R₂₅ are -OH, and both R₂₂ and R₂₆ are a hydrogen atom. This form corresponds to the formula (2d).
· 2,4,6-Trihydroxybenzoic acid: In the formula (2), R₂₁ is -COOH, all of R₂₂, R₂₄ and R₂₆ are -OH, and all of R₂₃ and R₂₅ are a hydrogen atom.

Another embodiment of the stabilizer represented by the formula (2) includes forms in which R₂₁ is -CH₂OH, -COOR₂₈, or -CONHR₂₈. Examples of the compound corresponding to this form include, but are not limited to, the following forms.
· Gentisyl alcohol: In the formula (2), R₂₁ is -CH₂OH, both R₂₂ and R₂₅ are - OH, and all of R₂₃, R₂₄ and R₂₆ are a hydrogen atom. This form corresponds to the formula (2e).
· Gentisic acid alkyl ester: In the formula (2), R₂₁ is -COOR₂₈, both R₂₂ and R₂₅ are -OH, all of R₂₃, R₂₄ and R₂₆ are a hydrogen atom, and R₂₈ is a saturated linear alkyl group having 1 or more and 8 or less carbon atoms. This form is a form included in the formula (2f).
· Gentisic acid ethanolamide: In the formula (2), R₂₁ is -CONHR₂₈, both R₂₂ and R₂₅ are -OH, all of R₂₃, R₂₄ and R₂₆ are a hydrogen atom, and R₂₈ is -CH₂-CH₂OH. This form is a form included in the formula (2g).

Among them, from the viewpoint of further increasing the yield of the radioactive metal complex, it is preferable to use a compound having a structure represented by any one of the formulas (2a) to (2g) or a salt thereof as the stabilizer, it is more preferable to use a compound having a structure represented by any one of the formulas (2a) to (2d) or a salt thereof, and it is still more preferable to use a compound having a structure represented by the formula (2b) or a salt thereof. That is, the stabilizer is more preferably salicylic acid, gentisic acid, protocatechuic acid or gallic acid or a salt thereof, still more preferably salicylic acid, gentisic acid or protocatechuic acid or a salt thereof, and most preferably gentisic acid or a salt thereof.

When a stabilizer is used from the viewpoint of further increasing the yield of the radioactive metal complex, the concentration of the stabilizer in the reaction liquid is preferably 0.1 mmol/L or more and 500 mmol/L or less, more preferably 1 mmol/L or more and 400 mmol/L or less, and still more preferably 1 mmol/L or more and 300 mmol/L or less at the start of the complex forming step. In addition, the concentration of the stabilizer in the reaction liquid is preferably higher than the concentration of the radioactive metal ions and the concentration of the ligand compound in the reaction liquid, from the viewpoint of preventing radiolysis and further improving the labeling efficiency.

In the present invention, it is not essential to add a stabilizer, but it is preferable to add a stabilizer when ⁸⁹Zr is used as the radioactive metal nuclide. This is because the use of ⁸⁹Zr is likely to cause a decrease in yield due to the adsorption described above.

The reaction liquid used in the complex forming step may not contain an organic solvent, and an organic solvent may be added depending on the physical properties of the ligand compound and the stabilizer. Examples of such an organic solvent include protic solvents such as methanol and ethanol, water-soluble aprotic solvents such as acetonitrile, N,N-dimethylformamide, tetrahydrofuran, dimethyl sulfoxide, and acetone, and the like. By containing such an organic solvent, even when a poorly water-soluble ligand compound and a poorly water-soluble stabilizer are used, these can be sufficiently dissolved or dispersed in the solvent, and a high labeling rate can be stably achieved.

The phrase "not contain an organic solvent" means that the organic solvent is not intentionally contained in the reaction liquid, but the organic solvent is allowed to be inevitably mixed in the reaction liquid.

The amount of the reaction liquid in the complex forming step is not particularly limited, but is practically 0.01 mL or more and 100 mL or less at the start of the complex forming step, from the viewpoint of practicality in the production step.

In the complex forming step, the order of addition of the radioactive metal source, the ligand compound, and other components is not limited as long as the labeling reaction of the radioactive metal ion to the ligand compound can proceed, specifically, the complex formation of the radioactive metal ion with the ligand compound is possible. For example, one of the radioactive metal source and the ligand compound may be added to a reaction vessel in which a mixed solvent prepared by mixing water, a stabilizer, and the second organic compound constituting the reaction liquid is stored in advance, and then the other may be added thereto to react them. Alternatively, to a solution in which one of the radioactive metal source and the ligand compound is dissolved in a mixed solvent, the other may be added to react them. Alternatively, the radioactive metal source and the ligand compound may be simultaneously added to a reaction vessel in which the mixed solvent is stored in advance to react them.

In the complex forming step, in order to further improve the labeling efficiency in a short reaction time, the reaction liquid is irradiated with microwave. In the specification of the present application, the microwave refers to an electromagnetic wave with a frequency of 10 MHz to 300 GHz. From the viewpoint of more effectively promoting the complex forming reaction, the frequency of the microwave to be irradiated is preferably 800 MHz or more and 3 GHz or less, and more preferably 2.35 GHz or more and 2.55 GHz or less.

As the microwave to be irradiated to the reaction liquid, for example, a microwave oscillated using a magnetron or semiconductor microwave generator is preferably used.

The output of the microwave to be irradiated is preferably 10 W or more, and more preferably 30 W or more. By setting the output of the microwave to 10 W or more, the reaction rate in the complex forming step can be effectively improved.

The microwave may be irradiated at a constant output during the complex forming step, or may be changed over time within the above range. Also, during the complex forming step, the microwave may be continuously or intermittently irradiated.

When the microwave is continuously irradiated, the irradiation time of the microwave can be set to the same time as the reaction time described later. When the microwave is intermittently irradiated, the total irradiation time of the microwave can be preferably 1 minute or more and 60 minutes or less, and more preferably 5 minutes or more and 30 minutes or less.

The temperature of the reaction liquid is preferably 10°C or more and 90°C or less, more preferably 30°C or more and 80°C or less, and still more preferably 50°C or more and 70°C or less, from the viewpoint of achieving both suppression of the decomposition of the ligand compound and further improvement of the labeling efficiency.

The reaction time is preferably 1 minute or more and 60 minutes or less, and more preferably 5 minutes or more and 30 minutes or less on condition that the reaction temperature is as described above.

In order to prevent the temperature of the reaction liquid from excessively increasing while maintaining the output of the microwave to be irradiated sufficiently high, in the present invention, the reaction liquid is irradiated with microwave while cooling the reaction liquid. In the specification of the present application, the "cooling of the reaction liquid" includes an embodiment in which the reaction liquid is directly cooled and an embodiment in which the reaction liquid is indirectly cooled by exposing a reaction vessel containing the reaction liquid to a cooling medium. From the viewpoint of simplicity of operation, it is preferable to indirectly cool the reaction liquid. In the embodiment in which the reaction liquid is indirectly cooled, the temperature of the reaction liquid is adjusted so as to be maintained in the above range by simultaneously performing heating of the reaction liquid by irradiation of microwave and cooling of the reaction liquid by a cooling medium.

The degree of cooling of the reaction liquid may be always constant during the complex forming step, or may be changed over time during the complex forming step.

Examples of the method of changing the degree of cooling of the reaction liquid over time include a method of changing the temperature of the cooling medium according to a predetermined schedule and a method of adjusting the temperature of the cooling medium according to the measured value of the temperature of the reaction liquid so that the temperature of the reaction liquid is maintained in an intended temperature range. Cooling can also be temporarily stopped.

Instead of changing the degree of cooling over time, the temperature of the reaction liquid may be maintained in the above range by changing the output of the microwave to be irradiated over time within the above range while keeping the degree of cooling constant. Alternatively, the temperature of the reaction liquid may be maintained in the above range by changing both the output of the microwave and the degree of cooling over time.

The irradiation of microwave and the cooling of the reaction liquid may be always performed during the complex forming reaction, or either one or both of them may be intermittently performed. However, from the viewpoint of enhancing the irradiation effect of microwave, it is preferable to always irradiate microwave during the complex forming reaction.

When the complex forming reaction is started, the irradiation of microwave may be started before the cooling of the reaction liquid, or the cooling of the reaction liquid may be started before the irradiation of microwave. Alternatively, both may be started simultaneously. From the viewpoint of preventing the temperature of the reaction liquid from excessively increasing, it is preferable to start cooling the reaction liquid first. However, when the temperature of the cooling medium is equal to or lower than the solidification point of the reaction solvent, it is preferable to start microwave irradiation immediately after the start of cooling in order to prevent the solidification of the reaction liquid.

Examples of the cooling medium used when the reaction liquid is indirectly cooled include a gas such as air, a liquid such as water or antifreeze liquid, and a solid such as an aluminum block. Among them, from the viewpoint of ease of temperature control, precision, and cooling efficiency, it is preferable to use air as a cooling medium, that is, to cool the reaction liquid by exposing the reaction vessel to cold air.

The temperature of the cooling medium is preferably -196°C or more and 90°C or less, more preferably -35°C or more and 25°C or less, and still more preferably -10°C or more and 0°C or less, from the viewpoint of facilitating the adjustment of the temperature of the reaction liquid.

As described in Examples described later, in the present invention, by irradiating a reaction liquid containing a stabilizer with microwave, the effect of improving the yield of a radioactive metal complex by irradiation of microwave can be further enhanced as compared with the case of irradiating a reaction liquid not containing a stabilizer with microwave.

In the complex forming step, the reaction is preferably performed in a state in which the pH of the reaction liquid is in an acidic region. That is, in the complex forming step, it is preferable to perform the reaction in a state where the acidic state of pH is maintained between the start and end of the reaction. The fact that the pH of the reaction liquid is in an acidic region means that the pH of the reaction liquid is less than 7. By performing the reaction in the state in which the pH of the reaction liquid is in an acidic region, the functional group of the ligand compound that interacts with the radioactive metal and/or the radioactive metal can be appropriately maintained in an ionic state to maintain a state in which they are easily coordinated to each other in the reaction liquid. As a result, the productivity of the radioactive metal complex can be further enhanced.

More specifically, the complex forming step is performed in a state in which the pH of the reaction liquid is preferably 2.0 or more and 6.0 or less.

The pH of the reaction liquid is adjusted in advance so as to be in an acidic region before the start of the reaction, that is, before the complex forming step is performed, whereby the pH of the reaction liquid can be maintained in the acidic region even during the complex forming step.

The pH of the reaction liquid can be adjusted, for example, by mixing an aqueous solution of the second organic compound and/or the stabilizer in the reaction liquid. In addition, the pH of the reaction liquid can be adjusted by preparing each of the radioactive metal ion-containing liquid, the aqueous solution of the ligand compound, the aqueous solution of the second organic compound, and the aqueous solution of the stabilizer in advance, and then adjusting the mixing ratio of these aqueous solutions. Alternatively, the pH of the reaction liquid can be adjusted by adding an inorganic acid such as hydrochloric acid or a metal hydroxide such as sodium hydroxide to a liquid obtained by mixing the radioactive metal ion, the ligand compound, the second organic compound, and the stabilizer.

In the complex forming step, it is preferable to set the amount of radioactivity of the radioactive metal nuclide in the reaction liquid at the start of the reaction as follows, from the viewpoint of improving the production efficiency of the radioactive metal complex.

In an embodiment in which ⁸⁹Zr is used as the radioactive metal nuclide, the amount of radioactivity of ⁸⁹Zr is 5 MBq or more, preferably 15 MBq or more, and more preferably 50 MBq or more as the amount of radioactivity in the reaction liquid at the start of the reaction in the complex forming step.

In an embodiment in which ²²⁵Ac is used as the radioactive metal nuclide, the amount of radioactivity of ²²⁵Ac is 1 MBq or more, preferably 2 MBq or more, and more preferably 4 MBq or more as the amount of radioactivity in the reaction liquid at the start of the reaction in the complex forming step.

In any embodiment, the upper limit of the amount of radioactivity in the reaction liquid at the start of the reaction is not particularly limited as long as it is an amount of radioactivity that can be realized on a commercial production scale, but can be, for example, 1,000 GBq or less.

In addition, in the complex forming reaction, it is preferable to set the ratio between the amount of radioactivity of the radioactive metal nuclide and the amount of the ligand compound in the reaction liquid at the start of the reaction to a value described below or more, from the viewpoint of reducing the amount of the ligand compound to be used and suppressing the production cost of the radioactive metal complex.

In the embodiment in which ⁸⁹Zr is used as the radioactive metal nuclide, the amount of radioactivity of the radioactive metal nuclide per 1 nmol of the ligand compound is preferably 10 MBq or more, more preferably 20 MBq or more, and still more preferably 60 MBq or more as the ratio between the ligand compound and the amount of radioactivity in the reaction liquid at the start of the reaction. The upper limit is not particularly limited, but is, for example, 10,000 MBq or less.

In the embodiment in which ²²⁵Ac is used as the radioactive metal nuclide, the amount of radioactivity of the radioactive metal nuclide per 1 nmol of the ligand compound is preferably 0.3 MBq or more, more preferably 1 MBq or more, and still more preferably 2 MBq or more as the ratio between the ligand compound and the amount of radioactivity in the reaction liquid at the start of the reaction. The upper limit is not particularly limited, but is, for example, 10,000 MBq or less.

In general, when the amount of radioactivity of the radioactive metal nuclide per 1 nmol of the ligand compound is increased, the yield of the complex forming reaction tends to decrease, but in the present invention, the complex forming reaction is effectively promoted by irradiation of microwave, and thus the complex forming reaction proceeds in high yield even when the amount of radioactivity of the radioactive metal nuclide per 1 nmol of the ligand compound is increased to the above range.

The reaction pressure in the complex forming step may be atmospheric pressure.

Since the production method of the present invention having the above-described complex forming step performs the complex forming reaction while irradiating microwave with a sufficiently high output, the progress rate of the complex forming reaction can be increased as compared with a conventional technique in which heating is performed without irradiating microwave or a conventional technique in which the irradiation condition of microwave is not optimized. This makes it possible to produce a radioactive metal complex in high yield even with a short reaction time. Also, in the production method of the present invention, by irradiating the reaction liquid with microwave while cooling the reaction liquid, it is possible to prevent the temperature of the reaction liquid from excessively increasing while increasing the output of the microwave to be irradiated. This makes it possible to suppress the progress of side reactions due to an excessive temperature rise of the reaction liquid.

Since the production method of the present invention has a high yield of the obtained radioactive metal complex, it is also advantageous in that the complex can be subjected to subsequent steps without separating and purifying unreacted radioactive metal nuclide.

In the formula (1), R₁₁, R₁₂, and R₁₃ are each preferably a carboxyalkyl group represented by a group consisting of -(CH₂)ₚCOOH and p is an integer of 1 or more and 3 or less from the viewpoint of improving the handleability of the ligand compound to be used and improving the stability of the ⁸⁹Zr complex to be obtained, particularly the stability of complex formation.

In this case, it is also preferable that one of R₁₄ and R₁₅ is a hydrogen atom or a carboxyalkyl group represented by a group consisting of -(CH₂)ₚCOOH, and p is an integer of 1 or more and 3 or less. Also, in this case, it is also preferable that the other of R₁₄ and R₁₅ is a carboxyalkyl group represented by a group consisting of -(CH₂)ₚCOOH and p is an integer of 1 or more and 3 or less, or a reactive atomic group for linking to a targeting agent or a group linking thereto.

When R₁₁, R₁₂, R₁₃, R₁₄, and R₁₅ have the above-described suitable groups, and when one of R₁₄ and R₁₅ is a hydrogen atom, the other of R₁₄ and R₁₅ is preferably a reactive atomic group for linking to a targeting agent or a group linking to a targeting agent.

That is, when R₁₄ is a reactive atomic group for linking to a targeting agent or a group linking to a targeting agent, R₁₅ is preferably a hydrogen atom, and when R₁₅ is a reactive atomic group for linking to a targeting agent or a group linking to a targeting agent, R₁₄ is preferably a hydrogen atom.

In the formula (1), when a ligand compound containing a group linking to a targeting agent is used, the targeting agent is preferably one or more of atomic groups including one selected from a low molecular weight compound, a polypeptide, a peptide aptamer, a growth factor, an affibody, a unibody, a nanobody, a monosaccharide, a polysaccharide, a vitamin, a nucleic acid, a liposome, a micelle, a carbon nanotube, or a nanoparticle. Among them, from the viewpoint of pharmaceutical use, the targeting agent is more preferably a low molecular weight compound, a polypeptide, or a nucleic acid.

The low molecular weight compound is, for example, a compound having a structure having target preference or a click-reactive atomic group.

The "targeting agent" in the present specification refers to a chemical structure for expressing directivity to a target organ or tissue in a living body or specificity to a target molecule. In the specification of the present application, a target organ or tissue or a target molecule is also collectively referred to as a "target site".

These targeting agents may be directly bonded to the ligand compound or indirectly bonded via another known linker structure such as PEG.

In addition, these targeting agents may be configured to be capable of being linked to a ligand compound using those conjugated with a reactive atomic group capable of binding to another structure. In order to link to the ligand compound, for example, a known reaction such as a click reaction can be adopted.

In a case where a click reaction is used for linking, for example, both the reactive atomic group of the targeting agent and the reactive atomic group for linking to a targeting agent of the ligand compound can be a group containing a click-reactive atomic group.

By using a ligand compound having such a chemical structure, it is possible to easily bind to a targeting agent having specificity or directivity for a target site, and it is possible to obtain a radioactive metal complex having specificity or directivity for a target site in high yield in a state in which the specificity or directivity for a target site of the targeting agent is sufficiently maintained.

When the targeting agent includes a polypeptide, the targeting agent is preferably a chain peptide, a cyclic peptide, or a combination thereof, or a protein, each of which specifically binds to a specific molecule. Examples of such an atomic group include peptides having three or more constituent amino acid residues. The molecular weight of the peptide is preferably 500 or more and 20,000 or less, and more preferably 1,000 or more and 6,000 or less, from the viewpoint of being able to be chemically controlled and synthesized.

Also, the polypeptide may be an antibody or a fragment thereof. Examples thereof include antibodies (immunoglobulins) having a class of IgG, IgA, IgM, IgD, and IgE, antibody fragments such as Fab fragments and F(ab')₂ fragments, peptide aptamers, and the like.

The amino acid constituting the above-described targeting agent may be a natural one or a synthetic one.

Various polypeptides that can be used as a targeting agent can be synthesized by conventionally known methods, for example, techniques such as liquid phase synthesis method, solid phase synthesis method, automatic peptide synthesis method, genetic recombination method, phage display method, genetic code reprogramming, and RaPID (Random non-standard Peptide Integrated Discovery) method. In the synthesis of various polypeptides, functional groups of amino acids to be used may be protected as necessary.

When the targeting agent is an atomic group containing a nucleic acid, the atomic group is preferably an atomic group containing an antisense nucleic acid, siRNA, miRNA, nucleic acid aptamer, decoy nucleic acid, cPG oligonucleic acid, or peptide nucleic acid, each of which specifically binds to a specific molecule. In addition, a nucleobase constituting such a targeting agent may be a natural one such as deoxyribonucleic acid or ribonucleic acid, or may be a synthetic one.

The atomic group containing the above-described nucleic acid that can be used in the present invention can be produced by a conventionally known method. For example, in the case of the nucleic acid aptamer, a nucleic acid aptamer that specifically binds to a specific target substance, such as a protein, can be produced using the SELEX method (Systematic Evolution of Ligands by Exponential Enrichment).

When a ligand compound containing a click-reactive atomic group is used as the ligand compound containing a reactive atomic group for linking to a targeting agent used in the present invention, the click-reactive atomic group derived from a known reagent that can be used for a click reaction can be appropriately used.

The "reactive atomic group" in the specification of the present application refers to a chemical structure in which a reaction when one compound and the other compound are bonded directly occurs. Examples of such a reactive atomic group include, but are not limited to, a click-reactive atomic group.

Examples of the click-reactive atomic group include an alkynyl group or an azido group, and a diene or a dienophile such as 1,2,4,5-tetrazine or an alkenyl group.

From the viewpoint of simplifying the click reaction step, the click-reactive atomic group as the reactive atomic group is preferably an atomic group that can be used for metal catalyst-free click reactions.

The click reaction is, for example, a reaction caused by a combination of an alkyne and an azide, or a combination of a diene and a dienophile, such as 1,2,4,5-tetrazine and an alkene. Specific examples of a click reaction by such a combination of atomic groups include Huisgen cycloaddition reaction, a Diels-Alder reaction, and the like.

Typically, the chemical structure produced by a click reaction in a combination of an alkyne and an azide contains a triazole skeleton, and the chemical structure produced by a click reaction in a combination of 1,2,4,5-tetrazine and an alkene as the combination of a diene and a dienophile contains a pyridazine skeleton. Therefore, when an atomic group containing an alkyne or an azide is contained as the click-reactive atomic group that can be contained in the reactive atomic group for linking to a targeting agent, a triazole skeleton can be formed by a click reaction. In addition, when an atomic group containing 1,2,4,5-tetrazine or an alkene which is a diene or a dienophile is contained as the click-reactive atomic group that can be contained in the reactive atomic group for linking to a targeting agent, a pyridazine skeleton can be formed by a click reaction.

Specific examples of the click-reactive atomic group include an atomic group containing dibenzocyclooctyne (DBCO) as an alkyne (formula (5a)), an atomic group containing an azido group as an azide (formula (5b)), an atomic group containing 1,2,4,5-tetrazine (formula (5c)), and an atomic group containing trans-cyclooctene (TCO) as an alkene (formula (5d)), as shown in the following formula.

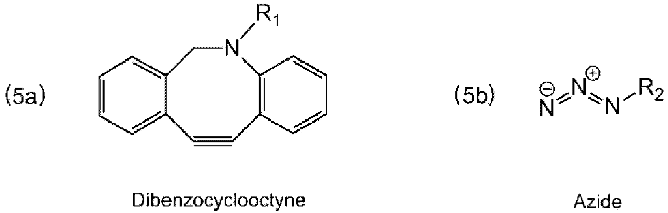

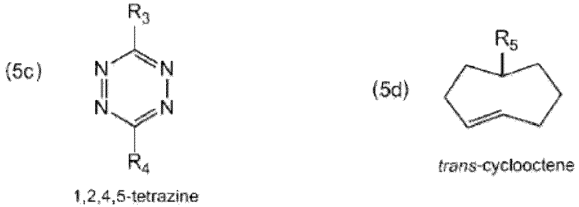

In the formula (5a), R₁ represents a binding site with an atomic group containing a ligand compound or a targeting agent.

In the formula (5b), R₂ represents a binding site with an atomic group containing a ligand compound or a targeting agent.

In the formula (5c), one of R₃ and R₄ represents a binding site with an atomic group containing a ligand compound or a targeting agent, and the other represents a hydrogen atom, a methyl group, a phenyl group, or a pyridyl group.

In the formula (5d), R₅ represents a binding site with an atomic group containing a ligand compound or a targeting agent.

When introducing a click-reactive atomic group into the ligand compound, it can be introduced using various commercially available reagents. Specifically, when introducing an atomic group containing dibenzocyclooctyne (DBCO) as a click-reactive atomic group, for example, DBCO reagents such as DBCO-C6-Acid, DBCO-Amine, DBCO-Maleimide, DBCO-PEG acid, DBCO-PEG-NHS ester, DBCO-PEG-Alcohol, DBCO-PEG-amine, DBCO-PEG-NH-Boc, Carboxyrhodamine-PEG-DBCO, Sulforhodamine-PEG-DBCO, TAMRA-PEG-DBCO, DBCO-PEG-Biotin, DBCO-PEG-DBCO, DBCO-PEG-Maleimide, TCO-PEG-DBCO, and DBCO-mPEG can be used.

As a suitable ligand compound used in the present invention, for example, a ligand compound having a structure represented by the following formulas (1-a) to (1-e) can be used, but the ligand compound is not limited thereto. The ligand compound having any structure sufficiently exhibits a stable effect of improving the labeling rate. In each of the following formulas, P represents an atomic group containing a reactive atomic group or an atomic group containing a targeting agent. From the viewpoint of stably improving the labeling rate, a ligand compound having a structure represented by the above formula (1-c) is more preferably used.

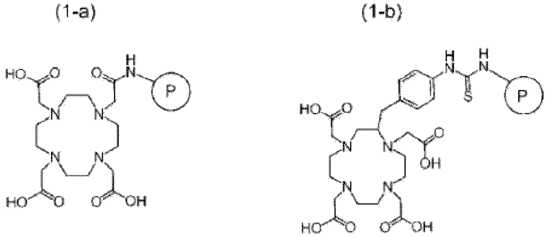

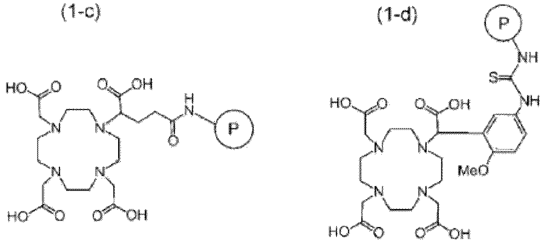

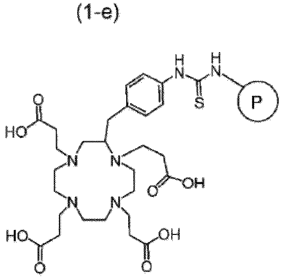

When a ligand compound containing a click-reactive atomic group is used in the formula (1), it is also preferable that the ligand compound and the click-reactive atomic group are indirectly bonded by a linker structure represented by the following formula (P). The structure is a structure derived from ethylene glycol, and in the formula (P), n is preferably an integer of 2 or more and 10 or less, and more preferably an integer of 2 or more and 8 or less.

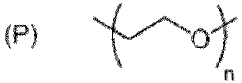

The structure of the ligand compound containing a click-reactive atomic group is not particularly limited as long as the effect of the present invention is exhibited, but it is more preferable to have the following structure. That is, the ligand compound more preferably has at least one of DO3A-DBCO, DOTA-DBCO, DO3A-PEG4-DBCO, DO4A-PEG7-Tz, and DOTAGA-DBCO shown below.

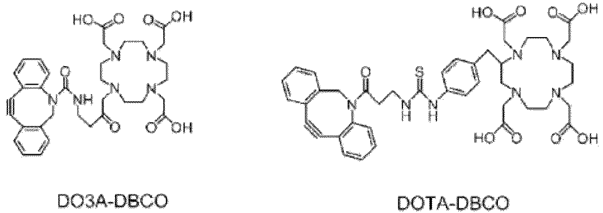

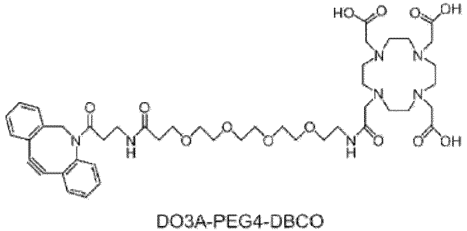

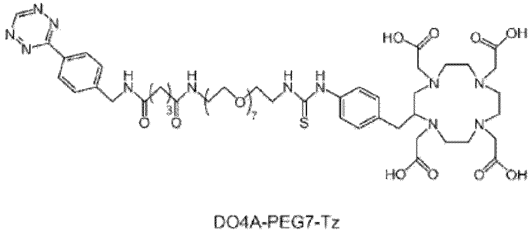

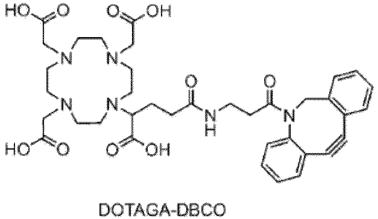

When a ligand compound containing a click-reactive atomic group is used as the reactive atomic group, for example, the ligand compound is coordinated to the radioactive metal nuclide by the above-described method, and then the click-reactive atomic group of the ligand compound and the click-reactive atomic group in the targeting agent are reacted by a click reaction or the like, whereby a radioactive metal complex can be produced.

In this case, as the targeting agent, a compound conjugated with a click-reactive atomic group that specifically binds to a reactive atomic group in the ligand compound can be used.

As the click-reactive atomic group to be conjugated with the targeting agent, the same group as one described above can be used. By using such a compound, a radioactive metal complex having specificity or directivity for a target site can be produced.

The radioactive metal complex produced through the above steps is present in a state of being dissolved in a reaction liquid. That is, the radioactive metal complex can be obtained as an aqueous liquid. The aqueous liquid containing the radioactive metal complex may be used as it is, or may be purified using a filtration filter, a membrane filter, a column packed with various fillers, chromatography, or the like.

Examples of a step after the radioactive metal complex is obtained include a formulation step for obtaining a radioactive drug containing the radioactive metal complex as an active ingredient. The formulation step can be performed by appropriately adding various stabilizers, such as a pH adjuster such as a citrate buffer, a phosphate buffer, or a borate buffer, a solubilizing agent such as polysorbate, a stabilizer, or an antioxidant, and diluting the mixture with an isotonic solution such as water or saline to adjust the concentration of radioactivity. In addition, after adding various stabilizers or adjusting the concentration as the formulation step, it is possible to include a step of performing sterile filtration with a membrane filter or the like to prepare an injectable preparation.

The above embodiments of the present invention include the following technical ideas.
[1] A method for producing a radioactive metal complex, including:
   a complex forming step of reacting a radioactive metal nuclide
   with a ligand compound represented by the above formula (1) in a reaction liquid containing water and a buffer to form a radioactive metal complex,
   the buffer containing one or more water-soluble organic compounds having a sulfo group or a carboxy group, and
   the reaction liquid being irradiated with microwave while the reaction liquid is cooled in the complex forming step.
[2] The production method according to [1], wherein the radioactive metal nuclide is ⁶⁸Ga, ⁸⁹Zr, ⁹⁰Y, ¹¹¹In, ¹⁷⁷Lu, or ²²⁵Ac.
[3] The production method according to [1] or [2], wherein the radioactive metal nuclide is ⁸⁹Zr.
[4] The production method according to any one of [1] to [3], wherein the reaction liquid contains a stabilizer.
[5] The production method according to [4], wherein the stabilizer is one or more selected from gentisic acid, salicylic acid, protocatechuic acid, and salts thereof.
[6] The production method according to any one of [3] to [5], wherein 10 MBq or more of the radioactive metal nuclide is used per 1 nmol of the ligand compound at the start of the complex forming step.
[7] The production method according to [2], wherein the radioactive metal nuclide is ²²⁵Ac.
[8] The production method according to [7], wherein 0.3 MBq or more of the radioactive metal nuclide is used per 1 nmol of the ligand compound at the start of the complex forming step.
[9] The production method according to any one of [1] to [8], wherein the targeting agent is a low molecular weight compound, a polypeptide, or a nucleic acid.
[10] The production method according to any one of [1] to [9], wherein the reaction liquid contains an organic solvent.
[11] The production method according to [10], wherein the organic solvent is one or more selected from acetonitrile, N,N-dimethylformamide, dimethyl sulfoxide, and ethanol.
[12] The production method according to any one of [1] to [11], wherein pH of the reaction liquid is in an acidic region.
[13] The production method according to any one of [1] to [12], wherein the water-soluble organic compound is one or more selected from acetic acid, phthalic acid, malonic acid, 2-[4-(2-hydroxyethyl)-1-piperazinyl]-ethanesulfonic acid, N-tris(hydroxymethyl)methyl-2-aminoethanesulfonic acid, 3-morpholinopropanesulfonic acid, and salts thereof.
[14] The production method according to any one of [1] to [13], wherein a temperature of the reaction liquid is 10°C or more and 90°C or less.
[15] The production method according to any one of [1] to [14], wherein in the complex forming step, the reaction liquid is irradiated with microwave at an output of 10 W or more.
[16] The production method according to any one of [1] to [15], wherein in the complex forming step, the reaction liquid is irradiated with microwave oscillated by using a magnetron or semiconductor microwave generator.
[17] The production method according to any one of [1] to [16], wherein in the complex forming step, a vessel containing the reaction liquid is cooled by being exposed to cold air.

### Examples

Hereinafter, the present invention will be described in more detail with reference to examples. However, the scope of the present invention is not limited to such examples. The following examples and comparative examples were all carried out at atmospheric pressure, and the reaction temperature of the complex forming reaction was 70°C. Also, the pH of the reaction liquid at the start of the reaction was 4.0 to 5.5 in all examples and comparative examples.

In the following examples and comparative examples, ⁸⁹Zr, DOTAGA-DBCO, and gentisic acid were used as a radioactive Zr element, a ligand compound, and a stabilizer, respectively. In addition, acetic acid and sodium acetate were used as second organic compounds.

⁸⁹Zr was taken out from bulk solutions of ⁸⁹Zr ions and used. The following lots A to D were used as bulk solutions of ⁸⁹Zr ions. Lot A: a bulk solution of 3.9 GBq of ⁸⁹Zr 4 days after production. Lot B: a bulk solution of 11.8 GBq of ⁸⁹Zr 1 day after production. Lot C: a bulk solution of 8.8 GBq of ⁸⁹Zr 4 days after production. Lot D: a bulk solution of 8.8 GBq of ⁸⁹Zr 6 days after production.

Since the reaction performance (labeling rate, adsorption rate, theoretical yield, and the like described later) of the complex forming reaction is affected by the lot of the bulk solution, it is necessary to compare the reaction performance of examples and comparative examples between examples and comparative examples using the same lot.

### [Example 1]

### (Preparation of reaction liquid)

A bulk solution of ⁸⁹Zr ions (lot A, solvent: 1.0 mol/L hydrochloric acid) was dispensed in an amount of 0.1 mL into a 2 mL vial, and the amount of radioactivity of the bulk solution was measured with a radio isotope dose calibrator (manufactured by CAPINTEC, Inc). The measured amount of radioactivity was 1,491 MBq.

Subsequently, the vial was heated at 110°C for 40 minutes under an argon stream to distill off the solvent. 100 µL of 0.1 mol/L hydrochloric acid was added to the obtained residue to prepare a ⁸⁹Zr ion-containing solution (radioactivity concentration: 14,910 MBq/mL) whose radioactivity concentration had been measured.

A part of the ⁸⁹Zr ion-containing solution (the amount required to adjust the charged radioactivity to 7.20 MBq) was added to a 10 mL glass tube, and 0.1 mol/L hydrochloric acid was further added to make 200 µL of a solution (hereinafter, it is also referred to as a "solution 1".).

Next, 200 µL of a 0.156 mol/L acetic acid-sodium acetate buffer containing gentisic acid at a concentration of 150 mmol/L (hereinafter, it is also referred to as a "solution 2".) and 200 µL of a 0.156 mol/L acetic acid-sodium acetate buffer containing DOTAGA-DBCO at a concentration of 0.01 mmol/L (hereinafter, it is also referred to as a "solution 3".) were added in this order, and the glass tube was stirred. The radioactivity of ⁸⁹Zr per 1 nmol (hereinafter, it is also referred to as "specific radioactivity".) of the ligand compound (DOTAGA-DBCO) contained in this reaction liquid was 3.6 MBq/nmol, and the amount of the reaction liquid was 600 µL.

### (Complex forming step)

Subsequently, a complex forming reaction was performed using a semiconductor microwave generator (MR-2G-100-CA50T15AC manufactured by Ryowa Electronics Co., Ltd.). First, cold air was blown to the glass tube, cooling of the reaction liquid in the glass tube was started, and immediately after that, irradiation of the reaction liquid with microwave was started. The maximum output of the microwave was set to 100 W, and during the complex forming reaction, the irradiation was continuously performed while the microwave output was varied so that the temperature of the reaction liquid was constant at 70°C. The temperature of the cold air was set to -5°C to -10°C, and the cold air in the temperature range was continuously blown to the glass tube during the complex forming reaction. After 10 minutes from the start of microwave irradiation, supply of cold air and microwave irradiation were stopped.

### (Analysis of reaction product)

2 µL of the reaction liquid was extracted and used for thin layer chromatography (TLC) analysis. TLC conditions: iTLC-SG (manufactured by Agilent), developing solvent: water/acetonitrile mixed solution (volume ratio 1:1)

The developed thin layer chromatogram was introduced into a TLC analyzer (GITAStar, manufactured by raytest), and the total ⁸⁹Zr radioactivity count containing unreacted ⁸⁹Zr and the radioactivity count of the ⁸⁹Zr complex in the reaction liquid were each measured. Then, the percentage of the radioactivity count of the ⁸⁹Zr complex with respect to the total ⁸⁹Zr radiation count was calculated as the labeling rate (%). The labeling rate indicates the degree of progress of the labeling reaction, and the higher the labeling rate, the more the intended ⁸⁹Zr complex is produced, which means that the labeling reaction proceeds well. The labeling rate of this sample was 91%.

In addition, the adsorption rate (%) of the ⁸⁹Zr complex was calculated on the basis of the adsorption amount confirmed by measuring the amount of radioactivity in the reaction vessel from which the reaction liquid was taken out with a radioisotope dose calibrator (manufactured by CAPINTEC, Inc.). The adsorption rate indicates the ratio of the radioactivity of ⁸⁹Zr adsorbed on the inner wall of the reaction vessel to the charged radioactivity. The adsorption rate of this sample was 19%.

Subsequently, the theoretical yield (%) of the ⁸⁹Zr complex was calculated by the following formula. Theoretical yield (%) = Labeling rate (%) × (100 - Adsorption rate (%))/100

The theoretical yield calculated by the above formula indicates the ratio of the ⁸⁹Zr complex that can be taken out from the reaction vessel and used to the amount of ⁸⁹Zr charged. The theoretical yield of this sample was 74%.

### [Examples 2 to 6, Comparative Examples 1 to 7]

⁸⁹Zr Complexes of Examples 2 to 6 and Comparative Examples 1 to 7 were produced in the same manner as in Example 1 except that the lot of the bulk solution of ⁸⁹Zr ions, the charged radioactivity, the addition amounts of the solutions 1 to 3, the concentration of the ligand compound DOTAGA-DBCO contained in the solution 3, the reaction time, and the heating method were set as shown in Table 1. The labeling rate, adsorption rate, and theoretical yield thereof are shown in Table 1.

In Comparative Examples 1 to 7, the reaction liquid was heated using a block heater without irradiation of microwave. The same applies to Comparative Examples 8 to 10 described later. Also, in Table 1 and Tables 2 and 4 described later, the semiconductor microwave generator is referred to as "Semiconductor MW".

**[Table 1]**

| | Solution 1 | Solution 2 | Solution 3 | | Reaction liquid amount (µL) | Zr lot | Heating method | Microwave maximum power (W) | Reaction time (min) | Charged radioactivity (MBq) | Ligand compound (nmol) | Specific radioactivity (MBq/nmol) | Labeling rate (%) | Adsorption rate (%) | Theoretical yield (%) |
|---|---|---|---|---|---|---|---|---|---|---|---|---|---|---|---|
| | Addition amount (µL) | Addition amount (µL) | Ligand compound concentration (mmol/L) | Addition amount (µL) | | | | | | | | | | | |
| Example 1 | 200 | 200 | 0.01 | 200 | 600 | A | Semiconductor MW | 100 | 10 | 7.20 | 2 | 3.60 | 91 | 19 | 74 |
| Example 2 | 200 | 200 | 0.005 | 200 | 600 | A | Semiconductor MW | 100 | 10 | 7.50 | 1 | 7.50 | 61 | 40 | 37 |
| Example 3 | 200 | 200 | 0.01 | 200 | 600 | A | Semiconductor MW | 100 | 10 | 28.5 | 2 | 14.3 | 70 | 34 | 46 |
| Example 4 | 200 | 200 | 0.01 | 200 | 600 | A | Semiconductor MW | 100 | 10 | 68.3 | 2 | 34.2 | 61 | 64 | 22 |
| Example 5 | 100 | 100 | 0.01 | 100 | 300 | B | Semiconductor MW | 100 | 10 | 81.0 | 1 | 81.0 | 90 | 18 | 74 |
| Example 6 | 100 | 100 | 0.01 | 100 | 300 | B | Semiconductor MW | 100 | 10 | 100 | 1 | 100 | 75 | 19 | 60 |
| Comparative Example 1 | 100 | 100 | 0.01 | 100 | 300 | A | Block heater | - | 10 | 3.44 | 1 | 3.44 | 48 | 17 | 39 |
| Comparative Example 2 | 100 | 100 | 0.01 | 100 | 300 | A | Block heater | - | 60 | 3.39 | 1 | 3.99 | 90 | 9 | 82 |
| Comparative Example 3 | 100 | 100 | 0.01 | 100 | 300 | A | Block heater | - | 10 | 7.18 | 1 | 7.18 | 14 | 24 | 11 |
| Comparative Example 4 | 100 | 100 | 0.01 | 100 | 300 | A | Block heater | - | 10 | 14.5 | 1 | 14.5 | 9 | 33 | 6 |
| Comparative Example 5 | 100 | 100 | 0.01 | 100 | 300 | A | Block heater | - | 10 | 34.3 | 1 | 34.3 | 12 | 19 | 10 |
| Comparative Example 6 | 100 | 100 | 0.01 | 100 | 300 | B | Block heater | - | 10 | 101 | 1 | 101 | 5 | 22 | 4 |
| Comparative Example 7 | 100 | 100 | 0.01 | 100 | 300 | B | Block heater | - | 60 | 108 | 1 | 108 | 39 | 25 | 29 |

As described in Table 1, comparing Examples 1 to 4, the lower the specific radioactivity, the higher the theoretical yield of the intended ⁸⁹Zr complex. In Examples 5 to 6, the bulk solution of ⁸⁹Zr ions of the lot having a small content of a non-radioactive metal nuclide as an impurity was used. In this case, although the specific radioactivity was as high as 81 to 100 MBq/nmol, a ⁸⁹Zr complex could be obtained in high theoretical yield. In Comparative Examples 1 to 7 in which the reaction liquid was heated with the block heater without irradiation of microwave, the theoretical yield was low as compared with Examples in which the specific radioactivity and the lot of the bulk solution of ⁸⁹Zr ions were the same. From this comparison, it is found that microwave irradiation has an effect of effectively promoting the complex forming reaction. In addition, it is found that a radioactive metal complex can be obtained in high yield even when the specific radioactivity is increased by irradiation of microwave.

Moreover, comparison between Example 1 in which the reaction was carried out for 10 minutes by irradiation of microwave and Comparative Example 2 in which the reaction was carried out for 60 minutes using the block heater shows that the labeling rate and the theoretical yield were comparable, and thus it is found that the reaction time could be shortened to about 1/6 by irradiation of microwave.

### [Examples 7 to 10, Comparative Example 8]

⁸⁹Zr Complexes of Examples 7 to 10 and Comparative Example 8 were produced in the same manner as in Example 1 except that the lot of the bulk solution of ⁸⁹Zr ions, the charged radioactivity, the addition amounts of the solutions 1 to 3, the concentration of the ligand compound DOTAGA-DBCO contained in the solution 3, and the heating method were set as described in Table 2. The labeling rate, adsorption rate, and theoretical yield thereof are shown in Table 2.

The magnetron microwave generator is referred to as "Magnetron MW" in Table 2 and Table 3 described later. In addition, as the magnetron microwave generator, Discover manufactured by CEM Corporation was used.

**[Table 2]**

| | Solution 1 | Solution 2 | Solution 3 | | Reaction liquid amount (µL) | Zr lot | Heating method | Microwave maximum power (W) | Reaction time (min) | Charged radioactivity (MBq) | Ligand compound (nmol) | Specific radioactivity (MBq/nmol) | Labeling rate (%) | Adsorption rate (%) | Theoretical yield (%) |
|---|---|---|---|---|---|---|---|---|---|---|---|---|---|---|---|
| | Addition amount (µL) | Addition amount (µL) | Ligand compound concentration (mmol/L) | Addition amount (µL) | | | | | | | | | | | |
| Example 7 | 200 | 200 | 0.01 | 200 | 600 | C | Semiconductor MW | 100 | 10 | 21.1 | 2 | 10.6 | 88 | 16 | 74 |
| Example 8 | 200 | 200 | 0.005 | 200 | 600 | C | Semiconductor MW | 100 | 10 | 21.7 | 1 | 21.7 | 80 | 18 | 66 |
| Example 9 | 200 | 200 | 0.01 | 200 | 600 | C | Magnetron MW | 100 | 10 | 25.5 | 2 | 12.8 | 93 | 4 | 89 |
| Example 10 | 200 | 200 | 0.005 | 200 | 600 | C | Magnetron MW | 100 | 10 | 26.6 | 1 | 26.6 | 69 | 6 | 64 |
| Comparative Example 8 | 100 | 100 | 0.01 | 100 | 300 | C | Block heater | - | 10 | 12.0 | 1 | 12.0 | 34 | 19 | 27 |

As shown in Table 2, the intended ⁸⁹Zr complex was obtained in a remarkably high theoretical yield as compared with Comparative Example 8 in which the complex forming reaction was performed using the block heater without irradiation of microwave, regardless of whether the semiconductor microwave generator (Examples 7 and 8) or the magnetron microwave generator (Examples 9 and 10) was used. In addition, since there is no large difference between the semiconductor microwave generator and the magnetron microwave generator, it is found that the reaction acceleration effect is almost the same regardless of which is used.

### [Examples 11 to 14, Comparative Examples 9 to 10]

⁸⁹Zr Complexes of Examples 11 to 14 and Comparative Examples 9 to 10 were produced in the same manner as in Example 1 except that the lot of the bulk solution of ⁸⁹Zr ions, the charged radioactivity, the addition amounts of the solutions 1 to 3, the reaction time, the heating method, and the maximum power of the microwave were set as shown in Table 3. The labeling rate, adsorption rate, and theoretical yield thereof are shown in Table 3.

**[Table 3]**

| | Solution 1 | Solution 2 | Solution 3 | | Reaction liquid amount (µL) | Zr lot | Heating method | Microwave maximum power (W) | Reaction time (min) | Charged radioactivity (MBq) | Ligand compound (nmol) | Specific radioactivity (MBq/nmol) | Labeling rate (%) | Adsorption rate (%) | Theoretical yield (%) |
|---|---|---|---|---|---|---|---|---|---|---|---|---|---|---|---|
| | Addition amount (µL) | Addition amount (µL) | Ligand compound concentration (mmol/L) | Addition amount (µL) | | | | | | | | | | | |
| Example 11 | 200 | 200 | 0.005 | 200 | 600 | D | Magnetron MW | 100 | 10 | 17.7 | 1 | 17.7 | 90 | 7 | 84 |
| Example 12 | 200 | 200 | 0.005 | 200 | 600 | D | Magnetron MW | 60 | 10 | 20.2 | 1 | 20.2 | 95 | 2 | 93 |
| Example 13 | 200 | 200 | 0.005 | 200 | 600 | D | Magnetron MW | 40 | 10 | 20 | 1 | 20 | 70 | 9 | 64 |
| Example 14 | 200 | 200 | 0.005 | 200 | 600 | D | Magnetron MW | 20 | 10 | 16.9 | 1 | 16.9 | 41 | 11 | 36 |
| Comparative Example 9 | 100 | 100 | 0.005 | 100 | 300 | D | Block heater | - | 10 | 10 | 0.5 | 20 | 18 | 19 | 15 |
| Comparative Example 10 | 100 | 100 | 0.005 | 100 | 300 | D | Block heater | - | 60 | 10 | 0.5 | 20 | 69 | 19 | 55 |

In Examples 11 to 14 in which the reaction was performed with the maximum output of the microwave set to 20 to 100 W, the intended ⁸⁹Zr complex was obtained in higher theoretical yield as compared with Comparative Example 9 in which heating was performed with the block heater without irradiation of microwave. The theoretical yield of the ⁸⁹Zr complex in the microwave was the highest particularly when the maximum output was 60 W. In addition, in Examples 11 to 13 in which the reaction was carried out for 10 minutes with the maximum output of the microwave being 40 to 100 W, higher theoretical yield was obtained than one in Comparative Example 10 in which the reaction was carried out for 60 minutes without irradiation of microwave, and thus it is found that the reaction time could be shortened to 1/6 or less under these reaction conditions as compared with the case where the microwave was not irradiated.

### [Examples 15 to 16]

A ⁸⁹Zr complex of Example 15 was produced in the same manner as in Example 1 except that the lot of the bulk solution of ⁸⁹Zr ions, the charged radioactivity, the addition amounts of the solutions 1 to 3, the heating method, and the maximum power of the microwave were set as shown in Table 4, and the ON/OFF of the microwave irradiation was switched every 10 seconds. Also, in Example 16, a ⁸⁹Zr complex of Example 16 was produced in the same manner as in Example 15 except that the solution 2 containing no gentisic acid as a stabilizer was used. The labeling rate, adsorption rate, and theoretical yield thereof are shown in Table 4.

**[Table 4]**

| | Solution 1 | Solution 2 | Solution 3 | | Reaction liquid amount (µL) | Zr lot | Heating method | Microwave maximum power (W) | Reaction time (min) | Charged radioactivity (MBq) | Ligand compound (nmol) | Specific radioactivit y (MBq/nmol ) | Stabilizer | Labeling rate (%) | Adsorption rate (%) | **Theoretical** yield (%) |
|---|---|---|---|---|---|---|---|---|---|---|---|---|---|---|---|---|
| | Addition amount (µL) | Addition amount (µL) | Ligand compound concentration (mmol/L) | Additio n amount (µL) | | | | | | | | | | | | |
| Example 15 | 100 | 100 | 0.01 | 100 | 300 | B | Semiconductor MW | 100 | 10 | 97 | 1 | 97 | Present | 49 | 23 | 38 |
| Example 16 | 100 | 100 | 0.01 | 100 | 300 | B | Semiconductor MW | 100 | 10 | 92 | 1 | 92 | Absent | 58 | 59 | 23 |

As shown in Table 4, it is found that Example 16 in which gentisic acid was not used has a higher adsorption rate and a lower theoretical yield as compared with Example 15 in which gentisic acid was used. That is, in the present invention, the effect of accelerating the complex forming reaction by irradiation of microwave and the effect of suppressing the adsorption of the radioactive metal complex by the stabilizer are combined, whereby the radioactive metal complex can be more efficiently produced.

## Claims

1. A method for producing a radioactive metal complex, comprising:
a complex forming step of reacting a radioactive metal nuclide
with a ligand compound represented by the following formula (1) in a reaction liquid containing water and a buffer to form a radioactive metal complex,
the buffer containing one or more water-soluble organic compounds having a sulfo group or a carboxy group, and
the reaction liquid being irradiated with microwave while the reaction liquid is cooled in the complex forming step, 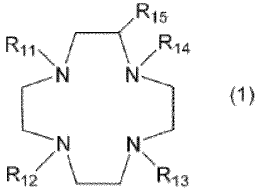
wherein R₁₁, R₁₂, and R₁₃ are each independently a group consisting of - (CH₂)ₚCOOH, -(CH₂)ₚC₅H₄N, -(CH₂)ₚPO₃H₂, or -(CH₂)ₚCONH₂, one of R₁₄ and R₁₅ is a group consisting of a hydrogen atom, -(CH₂)ₚCOOH, -(CH₂)ₚC₅H₄N, -(CH₂)ₚPO₃H₂, - (CH₂)ₚCONH₂ or -(CHCOOH)(CH₂)ₚCOOH, and the other is a group consisting of - (CH₂)ₚCOOH, -(CH₂)ₚC₅H₄N, -(CH₂)ₚPO₃H₂, or -(CH₂)ₚCONH₂, or a reactive atomic group for linking to a targeting agent or a group linking to a targeting agent, and each p is independently an integer of 0 or more and 3 or less.

2. The production method according to claim 1, wherein the radioactive metal nuclide is ⁶⁸Ga, ⁸⁹Zr, ⁹⁰Y, ¹¹¹In, ¹⁷⁷Lu, or ²²⁵Ac.

3. The production method according to claim 2, wherein the radioactive metal nuclide is ⁸⁹Zr.

4. The production method according to claim 3, wherein the reaction liquid contains a stabilizer.

5. The production method according to claim 4, wherein the stabilizer is one or more selected from gentisic acid, salicylic acid, protocatechuic acid, and salts thereof.

6. The production method according to claim 3 or 4, wherein 10 MBq or more of the radioactive metal nuclide is used per 1 nmol of the ligand compound at the start of the complex forming step.

7. The production method according to claim 2, wherein the radioactive metal nuclide is ²²⁵Ac.

8. The production method according to claim 7, wherein 0.3 MBq or more of the radioactive metal nuclide is used per 1 nmol of the ligand compound at the start of the complex forming step.

9. The production method according to claim 1 or 2, wherein the targeting agent is a low molecular weight compound, a polypeptide, or a nucleic acid.

10. The production method according to claim 1 or 2, wherein the reaction liquid contains an organic solvent.

11. The production method according to claim 10, wherein the organic solvent is one or more selected from acetonitrile, N,N-dimethylformamide, dimethyl sulfoxide, and ethanol.

12. The production method according to claim 1 or 2, wherein pH of the reaction liquid is in an acidic region.

13. The production method according to claim 1 or 2, wherein the water-soluble organic compound is one or more selected from acetic acid, phthalic acid, malonic acid, 2-[4-(2-hydroxyethyl)-1-piperazinyl]-ethanesulfonic acid, N-tris(hydroxymethyl)methyl-2-aminoethanesulfonic acid, 3-morpholinopropanesulfonic acid, and salts thereof.

14. The production method according to claim 1 or 2, wherein a temperature of the reaction liquid is 10°C or more and 90°C or less.

15. The production method according to claim 1 or 2, wherein in the complex forming step, the reaction liquid is irradiated with microwave at an output of 10 W or more.

16. The production method according to claim 1 or 2, wherein in the complex forming step, the reaction liquid is irradiated with microwave oscillated by using a magnetron or semiconductor microwave generator.

17. The production method according to claim 1 or 2, wherein in the complex forming step, a vessel containing the reaction liquid is cooled by being exposed to cold air.
